# EUROPEAN PATENT APPLICATION

(11) **EP 4 144 361 A1**
(43) Date of publication of application: **08.03.2023**
(21) Application number: 21780056.4
(22) Date of filing: 02.04.2021
(51) Int. Cl.: A61K 38/17, A61K 38/26, A61K 47/60, A61K 47/68, A61P 1/02, A61P 1/04, C07K 14/605

(54) **PHARMACEUTICAL COMPOSITION FOR PREVENTING OR TREATING MUCOSITIS INDUCED BY RADIOTHERAPY, CHEMOTHERAPY, OR COMBINATION THEREOF, COMPRISING GLP-2 DERIVATIVES OR LONG-ACTING CONJUGATE OF SAME**

(30) Priority: 03.04.2020 KR 20200040944
(71) Applicant: Hanmi Pharm. Co., Ltd., Hwaseong-si, Gyeonggi-do 18536 (KR)
(72) Inventor: LEE, Jin Bong, Hwaseong-si, Gyeonggi-do 18469 (KR); CHOI, Jae Hyuk, Hwaseong-si, Gyeonggi-do 18469 (KR); PARK, Eun Jin, Hwaseong-si, Gyeonggi-do 18469 (KR)
(74) Representative: Mewburn Ellis LLP
(86) International application number: PCT/KR2021/004162
(87) International publication number: WO 2021/201654

(57) **Abstract**

Disclosed is a prophylactic or therapeutic use of GLP-2 and a long-acting conjugate thereof for mucositis induced by radiotherapy, chemotherapy, or a combination thereof.

The GLP-2, the long-acting conjugate thereof, or the composition including the same of the present invention may be applied to preparation, treatment, and amelioration of mucositis induced by radiotherapy and/or chemotherapy.

## Description

### [Technical Field]

The present invention relates to a prophylactic or therapeutic use of a GLP-2 derivative and a long-acting conjugate thereof for mucositis induced by radiotherapy, chemotherapy, or a combination thereof.

### [Background Art]

Glucagon-like peptide-2 (GLP-2) is a peptide hormone consisting of 33 amino acids and produced by the small intestinal L cell in response to ingested nutrients. GLP-2 induces mucosal growth in the small and large intestines, promotes growth of intestinal cells and crypt cells, and inhibits apoptosis. In addition, GLP-2 increases absorption of nutrients in the small intestine and reduces intestinal permeability. Also, GLP-2 suppresses gastric emptying and secretion of gastric acid, increases intestinal blood flow velocity, and relaxes intestinal smooth muscles.

GLP-2 has shown promising potential as a therapeutic agent in various experimental models of intestinal diseases and intestinal injuries due to characteristics of energy absorption, protection, and activation of intestinal cell functions. As a hormone adjusting absorption of nutrients, GLP-2 has potential as a therapeutic agent for short bowel syndrome (SBS). SBS is a congenital disorder or acquired disorder induced by intestinal resection surgery and leads to nutrient deficiencies due to reduced absorption area in the small intestine. It was confirmed that GLP-2 enhances nutrient absorption and digestive tract absorption in an experimental model of rodents having SBS (Ljungmann K et al., Am. J. Physiol. Gastrointest Liver Physiol., 2001, 281(3):G779-85).

Meanwhile, during radiotherapy and/or chemotherapy, side effects such as mucositis occur because mucous membranes and the like, in which cells grow rapidly, are damaged as well as target cells (e.g., cancer cells). Therefore, there is a need to develop drugs capable of preventing or treating mucositis induced by radiotherapy and/or chemotherapy.

### [Disclosure]

### [Technical Problem]

An object of the present invention is to provide a composition for preventing, ameliorating, or treating mucositis induced by radiotherapy, chemotherapy, or a combination thereof, the composition comprising a glucagon-like peptide-2 (GLP-2) derivative and/or a long-acting conjugate thereof.

Another object of the present invention is to provide a method for preventing, ameliorating, or treating mucositis induced by radiotherapy, chemotherapy, or a combination thereof, the method comprising administering a GLP-2 derivative, a long-acting conjugate thereof, and/or a composition comprising the same to a subject in need thereof.

Another object of the present invention is to provide a use of a GLP-2 derivative, a long-acting conjugate thereof, and/or a composition comprising the same in preparation of a drug for preventing or treating mucositis induced by radiotherapy, chemotherapy, or a combination thereof.

### [Technical Solution]

An aspect of the present invention provides a composition for preventing, ameliorating, or treating mucositis induced by radiotherapy, chemotherapy, or a combination thereof, the composition comprising a glucagon-like peptide-2 (GLP-2) derivative and/or a long-acting conjugate thereof.

In a specific embodiment, the present invention provides a pharmaceutical composition for preventing or treating mucositis induced by radiotherapy, chemotherapy, or a combination thereof comprising a GLP-2 derivative and/or a long-acting conjugate thereof.

In another specific embodiment, the present invention provides a food composition for preventing or ameliorating mucositis induced by radiotherapy, chemotherapy, or a combination thereof comprising a GLP-2 derivative and/or a long-acting conjugate thereof.

The composition according to the specific embodiment of the present invention is a pharmaceutical composition for preventing or treating mucositis induced by radiotherapy, chemotherapy, or a combination thereof, the pharmaceutical composition comprising: a pharmaceutically acceptable excipient; and a glucagon-like peptide-2 (GLP-2) derivative in a pharmaceutically effective amount, wherein the GLP-2 derivative comprises an amino acid sequence represented by General Formula 1 below:

[General Formula 1] X₁X₂DGSFSDEMNTILDNLAARDFINWLIQTX₃₀ITDX₃₄ (SEQ ID NO: 10)

wherein
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or 2-aminoisobutyric acid (Aib);
X₃₀ is lysine or arginine;
X₃₄ is at least one amino acid or at least one altered amino acid; and
with the proviso that a sequence identical to SEQ ID NO: 1 is excluded from the amino acid sequence of General Formula 1.

The composition according to any one of the specific embodiments is characterized in that the alteration is substitution, addition, deletion, modification, or a combination thereof occurring at one or more amino acids.

The composition according to any one of the specific embodiments is characterized in that the GLP-2 derivative included an amino acid sequence represented by General Formula 2 below:

[General Formula 2] X₁X₂DGSFSDEMNTILDNLAARDFINWLIQTX₃₀ITDX₃₄ (SEQ ID NO: 9)

wherein
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or 2-aminoisobutyric acid (Aib);
X₃₀ is lysine or arginine;
X₃₄ is absent, or lysine, arginine, glutamine, histidine, 6-azidolysine, or cysteine; and
with the proviso that a sequence identical to SEQ ID NO: 1 is excluded from the amino acid sequence of General Formula 2.

The composition according to any one of the specific embodiments is characterized in that the GLP-2 derivative has a structure in which
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine.

The composition according to any one of the specific embodiments is characterized in that the GLP-2 derivative includes an amino acid sequence selected from the group consisting of SEQ ID NOS: 2 to 8.

The composition according to any one of the specific embodiments is characterized in that a C-terminus of the GLP-2 derivative is non-altered or amidated.

The composition according to any one of the specific embodiments is characterized in that the mucositis is oral mucositis, gastrointestinal mucositis, or a combination thereof.

The composition according to any one of the specific embodiments is characterized in that the chemotherapy is a chemotherapy using an anticancer drug.

The composition according to any one of the specific embodiments is characterized in that the anticancer drug is a cytotoxic anticancer agent, a targeted anticancer agent, an immuno-oncology agent, or a combination thereof.

The composition according to any one of the specific embodiments is characterized in that the cytotoxic anticancer agent is a nucleoside analogue, an antifolate, an antimetabolite, a Topoisomerase I inhibitor, an anthracycline, a podophyllotoxin, a taxane, a vinca alkaloid, an alkylating agent, a platinum compound, or a combination thereof.

The composition according to any one of the specific embodiments is characterized in that the anticancer drug is 5-fluorouracil (5-FU), cyclophosphamide (CPA), Docetaxel, Doxorubicin, Vincristine, Prednisone, Etoposide, ifosfamide, Methotrexate, Paclitaxel, Glemcitabine, Vinorelbine, Leucovorin, Irinotecan, Oxaliplatin, or a combination thereof.

The composition according to any one of the specific embodiments is characterized in that the composition is administered within 1 day before conducting radiotherapy or chemotherapy; or within 1 day after conducting radiotherapy or chemotherapy.

The composition according to any one of the specific embodiments is characterized in that the composition leads to at least one of an increase in a small intestine mass, a decrease in small intestine mass reduction level, an inhibition of inflammation, an inhibition of differentiation of monocytes into macrophages, and an inhibition of migration of monocytes, in a subject administered with the composition.

The composition according to any one of the specific embodiments is characterized in that the GLP-2 derivative is in the form of a long-acting conjugate in which the GLP-2 derivative is linked to a biocompatible material capable of increasing an *in vivo* half-life of the GLP-2 derivative.

The composition according to any one of the specific embodiments is characterized in that wherein the conjugate is represented by Chemical Formula 1 below:

[Chemical Formula 1] X-La-F

wherein,
X is a GLP-2 derivative;
L is a linker comprising an ethyleneglycol repeating unit;
a is 0 or a natural number with the proviso that when a is 2 or more, each L is independent of the other;
F is an immunoglobulin Fc region; and
the "-" is a covalent bond.

The composition according to any one of the specific embodiments is characterized in that the immunoglobulin Fc region is an aglycosylated IgG4 Fc region.

The composition according to any one of the specific embodiments is characterized in that the immunoglobulin Fc region includes a hinge region.

The composition according to any one of the specific embodiments is characterized in that the immunoglobulin Fc region is an IgG4 Fc region.

The composition according to any one of the specific embodiments is characterized in that the F is a dimer consisting of two polypeptide chains, and one end of the L is linked to only one polypeptide chain of the two polypeptide chains.

The composition according to any one of the specific embodiments is characterized in that the L is polyethylene glycol.

The composition according to any one of the specific embodiments is characterized in that a formula weight of the ethyleneglycol repeating unit moiety in the L is in a range of 1 kDa to 100 kDa.

Another aspect of the present invention provides a method for preventing, ameliorating, or treating mucositis induced by radiotherapy, chemotherapy, or a combination thereof, the method comprising administering a GLP-2 derivative, a long-acting conjugate thereof, and/or a composition comprising the same to a subject in need thereof, wherein the GLP-2 derivative comprises an amino acid sequence represented by General Formula 1 above (wherein a sequence identical to SEQ ID NO: 1 is excluded from the amino acid sequence of General Formula 1).

Another aspect of the present invention provides a prophylactic, ameliorative, or therapeutic use of a GLP-2 derivative, a long-acting conjugate thereof, or a composition comprising the same for mucositis induced by radiotherapy, chemotherapy, or a combination thereof, wherein the GLP-2 derivative includes an amino acid sequence represented by General Formula 1 above (wherein a sequence identical to SEQ ID NO: 1 is excluded from the amino acid sequence of General Formula 1).

Another aspect of the present invention provides a use of a GLP-2 derivative, a long-acting conjugate thereof, or a pharmaceutical composition comprising the same in preparation of a drug for preventing or treating mucositis induced by radiotherapy, chemotherapy, or a combination thereof, wherein the GLP-2 derivative includes an amino acid sequence represented by General Formula 1 above (wherein a sequence identical to SEQ ID NO: 1 is excluded from the amino acid sequence of General Formula 1).

### [Advantageous Effects]

The GLP-2 derivative, the long-acting conjugate thereof, or the composition including the same according to the present invention may be applied to prevention, treatment, and amelioration of mucositis induced by radiotherapy and/or chemotherapy.

### [Brief Description of Drawings]

FIG. 1 shows results of comparative analysis of changes in small intestine mass after administering a GLP-2 derivative long-acting conjugate to a rat model in which mucositis is induced by chemotherapy (Docetaxel and Cyclophosphamide, TC).
FIG. 2 shows results of comparative analysis of changes in small intestine mass by administering a GLP-2 derivative (Teduglutide) and a GLP-2 derivative long-acting conjugate simultaneously with chemotherapy (A); and by administering the GLP-2 derivative long-acting conjugate one day before chemotherapy in a rat model in which mucositis is induced by chemotherapy (TC).
FIG. 3 shows effects of a GLP-2 derivative long-acting conjugate on inhibiting M1 polarization (A), inhibiting macrophage differentiation (B), and inhibiting monocyte migration (C) in THP-1 cells (Monocytes).

### [Best Mode]

Hereinafter, the present invention will be described in detail.

Meanwhile, each of the descriptions and embodiments disclosed herein may be applied herein to describe different descriptions and embodiments. That is, all of the combinations of various factors disclosed herein belong to the scope of the present disclosure. Furthermore, the scope of the present invention should not be limited by the detailed descriptions provided hereinbelow.

Those skilled in the art will recognize or be able to ascertain, using no more than routine experimentation, many equivalents to specific embodiments of the present disclosure. Such equivalents are intended to be encompassed in the scope of the following claims.

Throughout the specification, not only the conventional one-letter and three-letter codes for naturally occurring amino acids, but also those three-letter codes generally allowed for other amino acids, such as α-aminoisobutyric acid (Aib) and 6-azidolysine (AZK) are used. In addition, the amino acids mentioned herein are abbreviated according to the nomenclature rules of the IUPAC-IUB as follows.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

An aspect of the present invention provides a composition for preventing, treating, or ameliorating mucositis induced by radiotherapy, chemotherapy, or a combination thereof, the composition comprising a glucagon-like peptide-2 (GLP-2) derivative. Specifically, the composition may be a pharmaceutical composition for preventing or treating mucositis induced by radiotherapy, chemotherapy, or a combination thereof.

Specific examples of the present invention may include a pharmaceutical composition for preventing or treating mucositis induced by radiotherapy, chemotherapy, or a combination thereof comprising a pharmaceutically acceptable excipient; and a GLP-2 derivative in a pharmaceutically effective amount, without being limited thereto.

As used herein, the term "mucositis" refers to inflammation and ulceration occurring in the mucous membranes of the oral cavity and/or the digestive tract, and the "mucositis induced by radiotherapy, chemotherapy, or a combination thereof" refers to mucositis induced as a side effect of radiotherapy or chemotherapy conducted alone or a combination of radiotherapy and chemotherapy regardless of the order or number of times, specifically oral mucositis, gastrointestinal mucositis, or a combination thereof, without being limited thereto. The gastrointestinal mucositis may be intestinal inflammatory disease and/or intestinal ulcer disease, but is not limited thereto. The gastrointestinal mucositis may cause symptoms such as nutrient deficiency or weight loss due to reduced nutrient absorption caused by diarrhea, anal ulcer, rectal ulcer, rectal bleeding, abdominal pain, dysphagia, vomiting, bloating, or decreases in villus height and crypt depth of the small intestine.

The radiotherapy refers to a therapy used to prevent or treat a disease using radiation beams and all types of radiotherapy used alone or in combination with chemotherapy and causing mucositis are included herein. In addition, the chemotherapy refers to a therapy used to prevent or treat a disease using a chemical medicine and all types of chemotherapy performed alone using a drug or in combination with radiotherapy and causing mucositis are included herein.

In a specific embodiment, the radiotherapy and chemotherapy may be used to prevent or treat a cancer, and the cancer may be lung cancer (e.g., non-small cell lung cancer, small cell lung cancer, or malignant mesothelioma), mesothelioma, pancreatic cancer (*e*.*g*., pancreatic duct cancer or pancreatic endocrine tumor), pharyngeal cancer, laryngeal cancer, esophageal cancer, gastric cancer (*e*.*g*., papillary adenocarcinoma, mucinous adenocarcinoma, or adenosquamous carcinoma), duodenal cancer, small intestine cancer, colorectal cancer (*e*.*g*., colon cancer, rectal cancer, anal cancer, familial colorectal cancer, hereditary nonpolyposis colorectal cancer, or gastrointestinal stromal tumor), breast cancer (*e*.*g*., invasive ductal cancer, non-invasive ductal cancer, or inflammatory breast cancer), ovarian cancer (*e*.*g*., epithelial ovarian carcinoma, extra-testicular germ cell tumor, ovarian germ cell tumor, or ovarian low grade serious tumor), testis cancer, prostate cancer (*e*.*g*., hormone-dependent prostate cancer or hormone-independent prostate cancer), liver cancer (*e*.*g*., hepatocellular carcinoma, primary liver cancer, or extrahepatic bile duct cancer), thyroid cancer (*e*.*g*., medullary thyroid carcinoma), kidney cancer (*e*.*g*., renal cell carcinoma or transitional cell carcinoma of the renal pelvis and ureter), uterine cancer (*e*.*g*., cervical cancer, cancer of uterine body, or uterine sarcoma), brain tumors (*e*.*g*., medulloblastoma, glioma, pineal astrocytoma, pilocytic astrocytoma, diffuse astrocytoma, anaplastic astrocytoma, or pituitary adenoma), retinoblastoma, skin cancer (*e*.*g*., basal cell carcinoma or malignant melanoma), sarcoma (*e*.*g*., rhabdomyosarcoma, leiomyosarcoma, or soft tissue sarcoma), malignant bone tumor, bladder cancer, blood cancer (*e*.*g*., multiple myeloma, leukemia, malignant lymphoma, Hodgkin's disease, or chronic myeloproliferative disease), or cancer of unknown primary, but is not limited thereto.

The chemotherapy for preventing or treating may be chemotherapy using an anticancer drug, and mucositis may be induced as a side effect thereof. The "using an anticancer drug" includes using an anticancer drug alone or in combination and using any other substance (*e*.*g*., drug other than the anticancer drug and drug inhibiting side effects of the anticancer drug) together with the anticancer drug concurrently, sequentially, or in reverse order to thereby induce mucositis.

In a specific embodiment, the anticancer drug inducing mucositis may be a cytotoxic anticancer agent, a targeted anticancer agent, an immuno-oncology agent, or a combination thereof, but is not limited thereto.

The cytotoxic anticancer agent is a drug attacking rapidly differentiating cells, specifically a nucleoside analogue (*e*.*g*., Azacitidine, Capecitabine, Carmofur, Cladribine, Clofarabine, Cytarabine, Decitabine, Floxuridine, Fludarabine, Fluorouracil, Gemcitabine, Mercaptopurine, Nelarabine, Pentostatin, Tegafur, and Tioguanine), an antifolate (*e*.*g*., Methotrexate, Pemetrexed, and Raltitrexed), an antimetabolite (*e*.*g*., hydroxyurea (Hydroxycarbamide)), a Topoisomerase I inhibitor (*e*.*g*., Irinotecan and Topotecan), an anthracycline (*e*.*g*., Daunorubicin, Doxorubicin, Epirubicin, and Idarubicin), a podophyllotoxin (*e*.*g*., Etoposide and Teniposide), a taxane (*e*.*g*., Cabazitaxel, Docetaxel, and Paclitaxel), a vinca alkaloid (*e*.*g*., Vinblastine, Vincristine, Vindesine, Vinflunine, and Vinorelbine), an alkylating agent (*e*.*g*., Bendamustine, Busulfan, Carmustine, Chlorambucil, Chlormethine, Cyclophosphamide, Dacarbazine, Fotemustine, Ifosfamide, Lomustine, Melphalan, Streptozotocin, and Temozolomide), a platinum compound (*e*.*g*., Carboplatin, Cisplatin, Nedaplatin, and Oxaliplatin), or any other drugs (*e*.*g*., Altretamine, Bleomycin, Bortezomib, Dactinomycin, Estramustine, Ixabepilone, Mitomycin, and Procarbazine), but is not limited thereto.

In addition, the targeted anticancer agent is a drug selectively attacking cancer cells having certain parts by targeting the certain parts different from other cells (*e*.*g*., normal cells), specifically a monoclonal antibody (*e*.*g*., Alemtuzumab, Bevacizumab, Cetuximab, Denosumab, Gemtuzumab ozogamicin, Ibritumomab tiuxetan, Ipilimumab, Nivolumab, Ofatumumab, Panitumumab, Pembrolizumab, Pertuzumab, Rituximab, Tositumomab, and Trastuzumab), a tyrosine kinase inhibitor (*e*.*g*., Afatinib, Aflibercept, Axitinib, Bosutinib, Crizotinib, Erlotinib, Gefitinib, Imatinib, Lapatinib, Nilotinib, Pazopanib, Ponatinib, Regorafenib, Ruxolitinib, Sorafenib, Sunitinib, and Vandetanib), an mTOR inhibitor (*e*.*g*., Everolimus and Temsirolimus), a retinoid (*e*.*g*., Alitretinoin, Bexarotene, Isotretinoin, Tamibarotene, and Tretinoin), an immunomodulator (*e*.*g*., Lenalidomide, Pomalidomide, and Thalidomide), a histone deacetylase inhibitor (Panobinostat, Romidepsin, Valproate, and Vorinostat), or any other drugs (Anagrelide, Arsenic trioxide, Asparaginase, BCG vaccine, Denileukin diftitox, and Vemurafenib, but is not limited thereto.

In addition, the immuno-oncology agent is a drug attacking cancer cells using the human immune system, specifically a drug that prevents cancer cells from evading the immune system or assists the immune cells to recognize and attack cancer cells more easily, more specifically an immune checkpoint inhibitor (*e*.*g*., Atezolizumab, Ipilimumab, Avelumab, Nivolumab, Pembrolizumab, and Durvalumab), an immune cell action enhancer (*e*.*g*., blinatumomab), or any other drugs (*e*.*g*., Alemtuzumab, Ofatumumab, and Elotuzumab), but is not limited thereto.

In addition, one drug may serve as a targeted anticancer agent and an immuno-oncology agent.

Also, in a specific embodiment, the anticancer drug that may cause mucositis may be 5-fluorouracil (5-FU), Cyclophosphamide (CPA), Docetaxel, Doxorubicin, Vincristine, Prednisone, Etoposide, ifosfamide, Methotrexate, Paclitaxel, Glemcitabine, Vinorelbine, Leucovorin, Irinotecan, Oxaliplatin, or a combination thereof, but is not limited thereto.

In a specific embodiment, the composition of the present invention may prevent or treat mucositis induced by radiotherapy, chemotherapy, or a combination thereof by leading to at least one of an increase in small intestine mass, inhibition of small intestine mass reduction (*i*.*e*., decrease in a small intestine mass reduction level), inhibition of inflammation, inhibiting differentiation of monocytes into macrophages, and inhibition of monocyte migration, in a subject administered with the composition, but is not limited thereto.

As used herein, the term "glucagon-like peptide-2" or "GLP-2" may be in the form of a polypeptide or a long-acting conjugate in which a biocompatible material capable of increasing an *in vivo* half-life thereof linked thereto as a glucagon-like peptide-2 receptor agonist, without being limited thereto. In the present invention, GLP-2 includes not only native (*e*.*g*., human) GLP-2, but also derivatives thereof and conjugates thereof. The GLP-2, as an active ingredient contained in the pharmaceutical composition of the present invention, may be contained in the pharmaceutical composition in a pharmaceutically effective amount.

In the present invention, the "GLP-2 receptor agonist" refers to a substance binding to a human glucagon-like peptide-2 receptor, *in vivo* or isolated, to induce physiological activity identical or similar to that of the native GLP-2. For example, the GLP-2 agonist may include native GLP-2 or a GLP-2 derivative.

The amino acid sequence of native GLP-2 is as follows.
GLP-2 (1-33)
HADGSFSDEMNTILDNLAARDFINWLIQTKITD (SEQ ID NO: 1)

In the present invention, the "GLP-2 derivative" includes a peptide having at least one amino acid different from an amino acid sequence of the native GLP-2 and a peptide obtained by altering the amino acid sequence of the native GLP-2 by modification while having a function of preventing, treating, or ameliorating mucositis induced by radiotherapy, chemotherapy, or a combination thereof; and/or a mimic of native GLP-2 having a function of preventing, treating, and/or ameliorating mucositis induced by radiotherapy, chemotherapy, or a combination thereof like native GLP-2.

Specifically, the GLP-2 derivative of the present invention may be obtained by alteration, selected from the group consisting of substitution, addition, deletion, modification and any combination, of at least one amino acid of the native GLP-2, without being limited thereto. In this case, an added amino acid may also be a non-native amino acid (*e*.*g*., D-amino acid), and the amino acid may be substituted with a non-native amino acid as well as a native amino acid. The amino acid sequence to which an amino acid is added may be derived from native GLP-2, but is not limited thereto. Also, in the present invention, alteration of an amino acid may mean that some groups of an amino acid residue are chemically substituted (*e*.*g*., α-methylation, α-hydroxylation, or substitution with an azido group), deleted (*e*.*g*., deamination), and/or modified (*e*.*g*., *N*-methylation) independently or together with the substitution, addition, deletion, or a combination thereof of at least one amino acid, without being limited thereto.

In a specific embodiment, the GLP-2 derivative of the present invention may have a homology (or identity) of 80%, 85%, 90%, 95%, 96%, 97%, 98%, 99%, or more with the amino acid sequence of the native GLP-2, and/or some groups of one amino acid residue of the GLP-2 derivative may be chemically substituted (*e*.*g*., α-methylation, α-hydroxylation, or substitution with an azido group), deleted (*e*.*g*., deamination), and/or modified (*e*.*g*., *N*-methylation), without being limited thereto.

In a specific embodiment, an N-terminal amino group of the GLP-2 derivative may be substituted, deleted, or modified, without being limited thereto. The GLP-2 derivative of the present invention may be prepared by a method of removing an α-amino group of an N-terminal histidine, a method of substituting an N-terminal amino group with an hydroxyl group or a carboxyl group, a method of remaining an imidazo-acetyl functional group by deleting an α-carbon of an N-terminal histidine and an N-terminal amino group linked to the α-carbon, a method of modifying an N-terminal amino group with two methyl groups, or the like, to inhibit linked to the N-terminus that is an important site for *in vivo* activity of the GLP-2 derivative in preparation of a long-acting conjugate.

Specifically, the GLP-2 derivative may be imidazoacetyl-deshistidyl GLP-2 (CA-GLP-2) from which α-carbon of a histidine residue, which is the first amino acid of the N-terminus, and an N-terminal amino group linked to the α-carbon, are deleted, desaminohistidyl GLP-2 (DA-GLP-2) in which the N-terminal amino acid group of GLP-2 is deleted, β-hydroxyimidazopropionyldeshistidyl GLP-2 (HY-GLP-2) in which the N-terminal amino acid of GLP-2 is substituted with a hydroxyl group, *N-*dimethylhistidyl GLP-2 (DM-GLP-2) in which the N-terminal amino group of GLP-2 is modified with 2 methyl groups, or β-carboxyimidazopropionyl-deshistidyl GLP-2 (CX-GLP-2) derivative in which the N-terminal amino group of GLP-2 is substituted with a carboxyl group, but is not limited thereto. As a non-limiting example, structures of substances used to prepare the GLP-2 derivative are as follows.

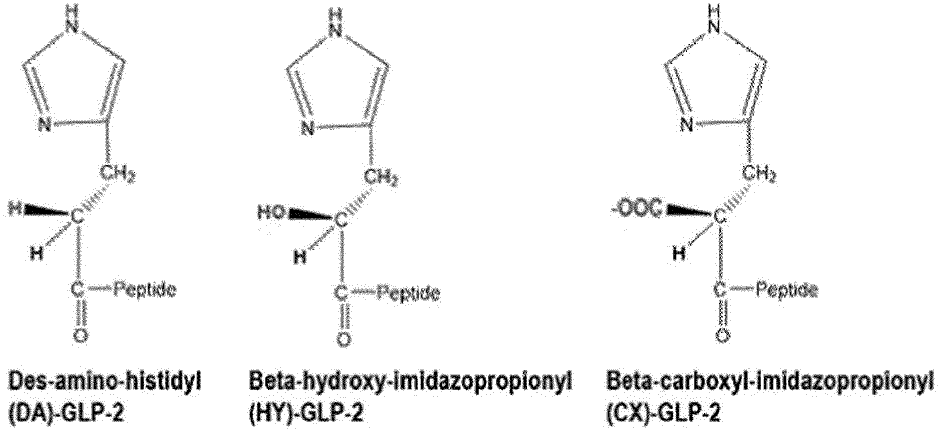

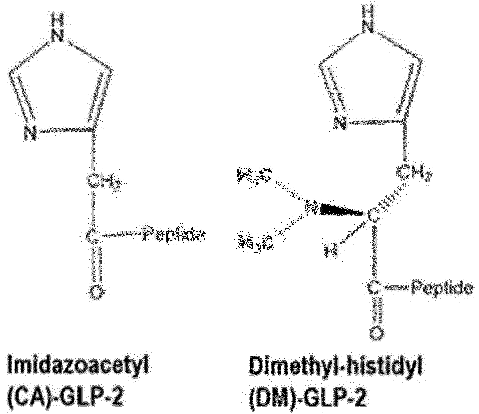

In a specific embodiment, the GLP-2 derivative may include alteration of at least one of 1^{st}, 2^{nd}, 30^{th}, and 33^{rd} amino acids in SEQ ID NO: 1, without being limited thereto. Specifically, in the present invention, alteration of an amino acid may be alteration, selected from the group consisting of substitution, addition, deletion, modification, and a combination thereof, of at least one amino acid. In this regard, an added amino acid may also be a non-native amino acid (*e*.*g*., D-amino acid), and the at least one amino acid may be substituted with a non-native amino acid as well as a native amino acid. An amino acid sequence including the added amino acid may be derived from native GLP-2, but is not limited thereto. In addition, the alteration of the present invention may mean that some groups of an amino acid residue are chemically substituted (*e*.*g*., α-methylation, α-hydroxylation, or substitution with an azido group), deleted (*e*.*g*., deamination), and/or modified (*e*.*g*., *N*-methylation) independently or together with the substitution, addition, deletion, or a combination thereof of at least one amino acid, without being limited thereto.

In a specific embodiment, the GLP-2 derivative may include an amino acid sequence of General Formula 1 below, without being limited thereto:

[General Formula 1] X₁X₂DGSFSDEMNTILDNLAARDFINWLIQTX₃₀ITDX₃₄ (SEQ ID NO: 10)

Herein,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or 2-aminoisobutyric acid (Aib);
X₃₀ is lysine or arginine;
X₃₄ is at least one amino acid or at least one altered amino acid; and
with the proviso that a sequence identical to SEQ ID NO: 1 is excluded from the amino acid sequence of General Formula 1.

Specifically, the amino acid may be a native amino acid or non-native amino acid, and the alteration is as described above.

In a specific embodiment, the GLP-2 derivative may include an amino acid sequence of General Formula 2 below, without being limited thereto:

[General Formula 2] X₁X₂DGSFSDEMNTILDNLAARDFINWLIQTX₃₀ITDX₃₄ (SEQ ID NO: 9)

Herein,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or 2-aminoisobutyric acid (Aib);
X₃₀ is lysine or arginine;
X₃₄ is absent, or lysine, arginine, glutamine, histidine, 6-azidolysine, or cysteine; and
with the proviso that a sequence identical to SEQ ID NO: 1 is excluded from the amino acid sequence of General Formula 2.

Specifically, the GLP-2 derivative of the present invention may include substitution of alanine, the 2^{nd} amino acid of the native GLP-2, with glycine or 2-aminoisobutyric acid (Aib), substitution of lysine, the 30^{th} amino acid, with arginine, or a combination thereof, without being limited thereto. In addition, the GLP-2 derivative may include a thiol group (*e*.*g*., cysteine), an amino group (*e*.*g*., lysine, arginine, glutamine, or histidine), or an azide group (*e*.*g*., 6-azidolysine), introduced into the C-terminus (*e*.*g*., the 33^{rd} amino acid), without being limited thereto.

Since linkage occurs at the introduced group during preparation of a long-acting conjugate of a GLP-2 derivative, a GLP-2 conjugate in which a binding site is selectively adjusted using the same may be prepared. Specifically, one end of a non-peptidyl linker may be linked to the hydroxyl group, the thiol group, the amino group, or the azide group of the GLP-2 derivative and the other end of the non-peptidyl linker may be linked to a substance capable of increasing an *in vivo* half-life (e.g., immunoglobulin Fc region). The thiol group, the amino group, or the azide group may be introduced by adding an amino acid to GLP-2, without being limited thereto. The thiol group may be introduced by adding cysteine (C) to GLP-2; the amino group may be introduced by adding lysine (K), arginine (R), glutamine (Q), or histidine (H) thereto; and the azide group may be introduced by adding 6-azidolysine (_{AZ}K) thereto, without being limited thereto.

Specifically, at least one residue of the GLP-2 derivative may be cysteine, lysine, arginine, glutamine, histidine, or 6-azidolysine, without being limited thereto.

Specifically, the GLP-2 derivative of the present invention may include substitution of alanine, the 2^{nd} amino acid of native GLP-2, with glycine and introduction of a thiol group (*e*.*g*., cysteine) into the C-terminus, more specifically include imidazoacetyldeshistidine from which α-carbon of a histidine residue, the first amino acid of the N-terminus, and an N-terminal amino group linked to the α-carbon, are deleted, thereby having an amino acid sequence of SEQ ID NO: 2, without being limited thereto.

Specifically, the GLP-2 derivative of the present invention may include substitution of alanine, the 2^{nd} amino acid of native GLP-2, with glycine and introduction of an amino group (*e*.*g*., lysine) into the C-terminus, more specifically include imidazoacetyldeshistidine from which α-carbon of a histidine residue, the first amino acid of the N-terminus, and an N-terminal amino group linked to the α-carbon, are deleted, thereby having an amino acid sequence of SEQ ID NO: 3, without being limited thereto.

Specifically, the GLP-2 derivative of the present invention may include substitution of alanine, the 2^{nd} amino acid of native GLP-2, with glycine, substitution of lysine, the 30^{th} amino acid of native GLP-2, with arginine, and introduction of an amino group (*e*.*g*., lysine) into the C-terminus, more specifically include imidazoacetyldeshistidine from which α-carbon of a histidine residue, the first amino acid of the N-terminus, and an N-terminal amino group linked to the α-carbon, are deleted, thereby having an amino acid sequence of SEQ ID NO: 4, without being limited thereto.

Specifically, the GLP-2 derivative of the present invention may include substitution of alanine, the 2^{nd} amino acid of native GLP-2, with glycine and introduction of an azide group (*e*.*g*., 6-azidolysine) into the C-terminus, more specifically include imidazoacetyldeshistidine from which α-carbon of a histidine residue, the first amino acid of the N-terminus, and an N-terminal amino group linked to the α-carbon, are deleted, thereby having an amino acid sequence of SEQ ID NO: 5, without being limited thereto.

Specifically, the GLP-2 derivative of the present invention may include substitution of alanine, the 2^{nd} amino acid of native GLP-2, with glycine, substitution of lysine, the 30^{th} amino acid of native GLP-2, with arginine and introduction of a thiol group (*e*.*g*., cysteine) into the C-terminus, more specifically include imidazoacetyldeshistidine from which α-carbon of a histidine residue, the first amino acid of the N-terminus, and an N-terminal amino group linked to the α-carbon, are deleted, thereby having an amino acid sequence of SEQ ID NO: 6, without being limited thereto.

Specifically, the GLP-2 derivative of the present invention may include substitution of alanine, the 2^{nd} amino acid of native GLP-2, with 2-aminoisobutyric acid and introduction of a thiol group (*e*.*g*., cysteine) into the C-terminus, thereby having an amino acid sequence of SEQ ID NO: 8, more specifically include imidazoacetyldeshistidine from which α-carbon of a histidine residue, the first amino acid of the N-terminus, and an N-terminal amino group linked to the α-carbon, are deleted, thereby having an amino acid sequence of SEQ ID NO: 7, without being limited thereto.

The GLP-2 derivatives having SEQ ID NOS: 2 to 8 are shown in Table 1 below.

**Table 1**

| **NAME** | SEQUENCE | SEQ ID NO |
|---|---|---|
| CA GLP-2 KC | *_{ca}*HGDGSFSDEMNTILDNLAARDFINWLIQTKITDC | 2 |
| CA GLP-2 KK | *_{ca}*HGDGSFSDEMNTILDNLAARDFINWLIQTKITDK | 3 |
| CA GLP-2 RK | *_{ca}*HGDGSFSDEMNTILDNLAARDFINWLIQTRITDK | 4 |
| CA GLP-2 K_{AZ}K | *_{ca}*HGDGSFSDEMNTILDNLAARDFINWLIQTKITD_{AZ}K | 5 |
| CA GLP-2 RC | *_{ca}*HGDGSFSDEMNTILDNLAARDFINWLIQTRITDC | 6 |
| CA GLP-2 Aib | *_{ca}*HAibDGSFSDEMNTILDNLAARDFINWLIQIKITDC | 7 |
| GLP-2 Aib | HAibDGSFSDEMNTILDNLAARDFINWLIQTKITDC | 8 |

In Table 1, _{ca}H indicates imidazoacetyldeshistidine substituted instead of histidine, Aib indicates 2-aminoisobutyric acid, and _{AZ}K indicates 6-azido-L-lysine.

The GLP-2 derivative according to the present invention may be a peptide including the above-described sequence, or a peptide (essentially) consisting of the above-described sequence, but is not limited thereto.

Although described as a peptide or GLP-2 derivative "consisting of a particular SEQ ID NO" in the present invention, it does not exclude a mutation that may occur naturally or by addition of a meaningless sequence upstream or downstream of the amino acid sequence of the SEQ ID NO, or a silent mutation thereof, as long as the peptide or GLP-2 derivative has activity identical or equivalent to that of the peptide or GLP-2 derivative consisting of the amino acid sequence, and even when such sequence addition or mutation is present, it obviously belongs to the scope of the present invention.

Specifically, the GLP-2 derivative may be a compound of General Formula 1 or 2 in which (1) X₂ is glycine or Aib, (2) X₃₀ is lysine or arginine, or (3) X₂ is glycine or Aib, and X₃₀ is lysine or arginine, without being limited thereto.

Specifically, the GLP-2 derivative may be a compound of General Formula 1 or General Formula 2, in which
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine, without being limited thereto.

In the present invention, such alteration for the preparation of the agonist, fragment, variant, and derivative the native GLP-2 includes alteration using L-or D-amino acids and/or non-native amino acids; and/or alteration of a native sequence after modification or translation (*e*.*g*., methylation, acylation, ubiquitination, and intramolecular covalent bonding).

In the present invention, the GLP-2 derivative may be in an altered form where the N-terminus and/or C-terminus is chemically modified or protected by organic groups, or amino acids may be added to the termini of the GLP-2 derivative, for protection from proteases *in vivo* while increasing stability thereof.

Particularly, since the N- and C-termini of chemically-synthesized peptides are electrically charged, the N-terminus may be acetylated and/or the C-terminus may be amidated to remove the charges, but the embodiment is not limited thereto. Specifically, in the present invention, the GLP-2 derivative may have a C-terminus non-altered or aminated, without being limited thereto.

The GLP-2 derivative of the present invention may be synthesized via solid phase synthesis, recombination, or commercially purchased.

In the present invention, the GLP-2 derivative may be in the form of a long-acting conjugate thereof in which a biocompatible material capable of increasing an *in vivo* half-life thereof is linked to the GLP-2 derivative, without being limited thereto. The long-acting conjugate may have long-lasting efficacy compared to the GLP-2 derivative to which the biocompatible material (*e*.*g*., immunoglobulin Fc region) is not linked. In the present invention, the conjugate including the biocompatible material linked to the GLP-2 derivative and having an increased half-life of the GLP-2 derivative is referred to as "GLP-2 derivative long-acting conjugate" or "long-acting conjugate of the GLP-2 derivative". In the present invention, the long-acting conjugate may be used interchangeably with the conjugate.

Meanwhile, the conjugate may be a conjugate that does not occur naturally.

In addition, in the GLP-2 derivative long-acting conjugate, linkage between the GLP-2 derivative and the biocompatible material (*e*.*g*., immunoglobulin Fc region) may be formed by a physical or chemical bond, or a covalent or non-covalent bond, without being limited thereto.

In addition, although a method of linking the GLP-2 derivative to the biocompatible material (*e*.*g*., immunoglobulin Fc region) in the GLP-2 derivative long-acting conjugate is not particularly limited, the GLP-2 derivative may be linked to the biocompatible material (*e*.*g*., immunoglobulin Fc region) via a linker.

In addition, Korean Patent Application Laid-open Publication No. 10-2019-0037181 that discloses a GLP-2 derivative long-acting conjugate is incorporated herein by reference.

In a specific embodiment, the long-acting conjugate of the GLP-2 derivative of the present invention may have a structure represented by Chemical Formula 1 below, without being limited thereto.

[Chemical Formula 1] X-La-F

Herein,
X is the GLP-2 derivative disclosed in the present invention;
L is a linker (*e*.*g*., linker comprising an ethyleneglycol repeating unit);
a is 0 or a natural number with the proviso that when a is 2 or more, each L is independent of the other;
F is a biocompatible material (*e*.*g*., immunoglobulin Fc region) capable of increasing an *in vivo* half-life of X; and
the "-" is a chemical bond (*e*.*g*., covalent bond).

More specifically, X and L, and L and F may be linked to each other by a covalent bond. In this case, the conjugate may be a conjugate in which X, L, and F are linked by covalent bonds, respectively, in the order shown in Chemical Formula 1.

In addition, F may be directly linked to X (*i*.*e*., a is 0 in Chemical Formula 1) or via a linker (L).

In the conjugate according to the present invention, F that is a substance capable of increasing the half-life of X, *i*.*e*., the GLP-2 derivative according to the present invention, is a component of a moiety constituting the conjugate of the present invention.

F may be linked to X by a covalent chemical bond or a non-covalent chemical bond. Via the covalent chemical bond, the non-covalent chemical bond, or a combination thereof, F may be linked to X via L.

F, as a biocompatible material capable of increasing the half-life of X, may be, for example, selected from the group consisting of a polymer, fatty acid, cholesterol, albumin and fragments thereof, an albumin binding substance, a polymer of a repeating unit of a certain amino acid sequence, an antibody, an antibody fragment, an FcRn-binding substance, *in vivo* connective tissue, nucleotide, fibronectin, transferrin, saccharide, heparin, and elastin, but is not particularly limited thereto.

The elastin may be human tropoelastin that is a water-soluble precursor, a polymer of a partial sequence or a repeating unit, *e*.*g*., all elastin-like polypeptides, but is not particularly limited thereto.

Examples of the polymer may be a polymer selected from the group consisting of polyethylene glycol (PEG), polypropylene glycol, ethylene glycol-propylene glycol copolymer, polyoxyethylated polyol, polyvinylalcohol, polysaccharide (*e*.*g*., dextran), polyvinylethylether, biodegradable polymer, lipid polymer, chitin, hyaluronic acid, oligonucleotide, and a combination thereof. The polysaccharide may include dextran, but is not particularly limited thereto.

The polyethylene glycol is a term including all of an ethylene glycol homopolymer, a PEG copolymer, or a monomethyl-substituted PEG polymer (mPEG), without being limited thereto.

Also, the biocompatible material includes poly-amino acid such as poly-lysine, poly-aspartic acid, and poly-glutamic acid, without being limited thereto.

In addition, the fatty acid may have a binding affinity for albumin in a living body, without being limited thereto.

As an example of the F, the F may be an FcRn-binding substance. Specifically, the FcRn-binding substance may be an immunoglobulin Fc region, more specifically an IgG Fc region, even more specifically an aglycosylated IgG4 Fc region, but is not particularly limited thereto.

In an exemplary embodiment of the present invention, the F (*e*.*g*., immunoglobulin Fc region) may be a dimer consisting of two polypeptide chains and have a structure in which one end of L is linked to only one polypeptide chain of the two polypeptide chains, without being limited thereto.

In addition, L may be a peptidyl linker or a non-peptidyl linker (*e*.*g*., linker comprising an ethyleneglycol repeating unit).

When the L is a peptidyl linker, the peptidyl linker may include one or more amino acids, *e*.*g*., 1 to 1000 amino acids, without being limited thereto. Various known peptide linkers may be used to link F to X in the present invention and may include, for example, [GS]ₓ linker, [GGGS]ₓ linker, and [GGGGS]ₓ linker, where x is a natural number of 1 or greater. However, the present invention is not limited thereto.

In the present invention, the "non-peptidyl linker" includes a biocompatible polymer composed of two or more repeating units linked to each other. The repeating units are linked to each other via any covalent bond other than the peptide bond. In the present invention, the non-peptidyl linker includes a reactive group at one end and may form a conjugate via reaction with other components constituting the conjugate. The non-peptidyl linker may be a component constituting a moiety of the conjugate of the present invention.

The non-peptidyl linker available in the present invention is not limited as long as it has *in vivo* resistance to a protease.

In the present invention, the non-peptidyl linker may be used interchangeably with the non-peptidyl polymer.

Specifically, the non-peptidyl linker may be selected from the group consisting of a fatty acid, a saccharide, a polymer, a low-molecular weight compound, a nucleotide, and a combination thereof.

The polymer may be selected from the group consisting of polyethylene glycol, polypropylene glycol, ethylene glycol-propylene glycol copolymer, polyoxyethylated polyol, polyvinylalcohol, polysaccharide, polyvinylethylether, biodegradable polymer, lipid polymer, chitin, hyaluronic acid, oligonucleotide, and a combination thereof, and the polysaccharide may be dextran, without being limited thereto.

Also, although not particularly limited thereto, a molecular weight of the polymer of the present invention may be greater than 0 kDa and less than about 100 kDa, in the range of about 1 kDa to about 100 kDa, specifically about 1 kDa to about 20 kDa, without being limited thereto.

Although not particularly limited thereto, the non-peptidyl linker may be selected from the group consisting of polyethylene glycol, polypropylene glycol, ethylene glycol-propylene glycol copolymer, polyoxyethylated polyol, polyvinyl alcohol, polysaccharide (e.g., dextran), polyvinyl ethylether, biodegradable polymer such as polylactic acid (PLA) and polylactic-glycolic acid (PLGA), lipid polymer, chitin, hyaluronic acid, oligonucleotide, and a combination thereof.

Although not particularly limited thereto, the non-peptidyl linker may be a linker comprising an ethyleneglycol repeating unit, such as polyethylene glycol, and any derivatives thereof well known in the art and easily prepared at the level of the technology in the art belong to the scope of the present invention.

The repeating unit of the non-peptidyl linker may be an ethyleneglycol repeating unit, specifically, the non-peptidyl linker may include both the ethyleneglycol repeating unit and a functional group used for preparation of the conjugate. In the long-acting conjugate according to the present invention, X may be linked to F via the functional groups, without being limited thereto. In the present invention, the non-peptidyl linker may include two, three, or more functional groups, which may be the same or different, without being limited thereto.

Specifically, the linker may be polyethyleneglycol (PEG) represented by Chemical Formula 2 below, but is not limited thereto:

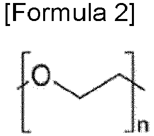

In Chemical Formula 2, n = 10 to 2400, n = 10 to 480, or n = 50 to 250, without being limited thereto.

In the long-acting conjugate, the PEG moiety may include not only a - (CH₂CH₂O)ₙ- structure and an oxygen atom interposed between a linking element and the -(CH₂CH₂O)ₙ- structure, without being limited thereto.

Also, in a specific embodiment, the conjugate may have a structure in which GLP-2 is linked to the immunoglobulin Fc region (F) via the linker including an ethylene glycol repeating unit by covalent bonds, without being limited thereto.

The polyethylene glycol is a term including all forms of an ethylene glycol homopolymer, a PEG copolymer, and a monomethyl-substituted PEG polymer (mPEG), but is not particularly limited thereto.

The non-peptidyl linker available in the present invention may be any polymer resistant to proteases *in vivo,* without limitation. A molecular weight of the non-peptidyl polymer may be greater than 0 kDa and less than about 200 kDa, specifically in the range of about 1 kDa to about 100 kDa, more specifically about 1 kDa to about 50 kDa, more specifically about 1 kDa to about 20 kDa, more specifically about 3.4 kDa to 10 kDa, even more specifically about 3.4 kDa, but is not limited thereto.

As used herein, the term "about" refers to a range including all of ±0.5, ±0.4, ±0.3, ±0.2, ±0.1, or the like and includes all numerical values equivalent to those which come immediately after the term "about" or those in a similar range, without being limited thereto.

In addition, as the non-peptidyl linker of the present invention linked to the immunoglobulin Fc region, not only one type of polymer but also a combination of different types of polymers may be used.

In a specific embodiment, both ends of the non-peptidyl linker may be linked to the thiol group, the amino group, or the hydroxyl group of the immunoglobulin Fc region and the thiol group, the amino group, the azide group, or the hydroxyl group of GLP-2, without being limited thereto.

Specifically, the non-peptidyl linker may include reactive groups at both ends respectively linked to the immunoglobulin Fc region and GLP-2 or a derivative thereof, specifically a reactive group linked to a thiol group of cysteine; an amino group of the N-terminus, lysine, arginine, glutamine and/or histidine; and or a hydroxyl group of the C-terminus of the immunoglobulin Fc region, and a reactive group linked to a thiol group of cysteine; an amino group of lysine, arginine, glutamine, and/or histidine; an azide group of azidolysine; and/or a hydroxyl group of the GLP-2, without being limited thereto.

More specifically, the reactive group of the non-peptidyl polymer may include at least one selected from the group consisting of an aldehyde group, a maleimide group, and a succinimide derivative, without being limited thereto.

In the above description, the aldehyde group may be a propionaldehyde group or a butyraldehyde group, without being limited thereto.

In the above description, the succinimide derivative may be succinimidyl carboxymethyl, succinimidyl valerate, succinimidyl methyl butanoate, succinimidyl methylpropionate, succinimidyl butanoate, succinimidyl propionate, *N-*hydroxysuccinimide, hydroxysuccinimidyl, or succinimidyl carbonate, without being limited thereto.

The non-peptidyl linker may be linked to the immunoglobulin Fc and the GLP-2 derivative via the above-described reactive groups to be converted into a non-peptidyl polymer linker.

Also, a final product produced by reductive amination by aldehyde bonds is more stable than that formed by an amide bond. The aldehyde reactive group selectively reacts with the N-terminus at a low pH while forming a covalent bond with a lysine residue at a high pH, *e*.*g*., at a pH of 9.0.

The reactive groups of both ends of the non-peptidyl linker may be the same or different. The non-peptidyl linker may include aldehyde reactive groups at the ends. Also, the non-peptidyl linker may include an aldehyde group and a maleimide reactive group at the ends or an aldehyde group and a succinimidyl reactive group at the ends, without being limited thereto.

For example, the non-peptidyl linker may include a maleimide group at one end and an aldehyde group, a propionaldehyde group, or a butyraldehyde group at the other end, as reactive groups. As another example, the non-peptidyl linker may include a succinimidyl group at one end and a propionaldehyde group or a butyr aldehyde group at the other end.

In the case where polyethylene glycol having a hydroxyl reactive group at the propion-containing end is used as the non-peptidyl linker, the conjugate of the present invention may be prepared by activating the hydroxy group using various reactive groups by known chemical reactions or using commercially available polyethylene glycol having modified reactive groups.

In a specific embodiment, the reactive group of the non-peptidyl linker may be linked to a cysteine residue, more specifically a -SH group of cysteine, of GLP-2, without being limited thereto.

In the case of using maleimide-PEG-aldehyde, the maleimide group may be linked to the -SH group of the GLP-2 derivative via a thioether bond and the aldehyde group may be linked to the -NH₂ group of the immunoglobulin Fc via reductive alkylation, but the embodiment is not limited thereto and this is merely an example.

The N-terminal amino group of the immunoglobulin Fc region may be linked to an oxygen atom located at one end of PEG by a linker functional group having a - CH₂CH₂CH₂- structure via reductive alkylation to form a -PEG-O-CH₂CH₂CH₂NH-immunoglobulin Fc structure, and the other end of PEG may be linked to a sulfur atom located at cysteine of GLP-2 via the thioether bond. The above-described thioether bond may include a structure of

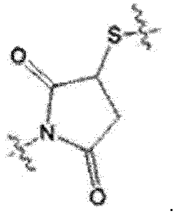

However, the embodiment is not limited thereto and this is merely an example.

In addition, in the conjugate, the reactive group of the non-peptidyl linker may be linked to the -NH₂ group of the N-terminus of the immunoglobulin Fc, but this is merely an example.

Also, in the conjugate, the GLP-2 derivative may be linked to the non-peptidyl linker having a reactive group via the C-terminus, but this is merely an example.

In the present invention, "C-terminus" refers to a carboxyl terminus of a peptide indicating a site used to be linked to a non-peptidyl polymer in view of the objects of the present invention. For example, the "C-terminus" may include not only the last amino acid residue of the C-terminus but also amino acid residues near the C-terminus, specifically amino acid residues up to the 20^{th} amino acid residue from the last amino acid.

In a specific embodiment, in the long-acting conjugate of the present invention, the GLP-2 derivative may be linked to the immunoglobulin Fc region, without being limited thereto.

As used herein, the term "immunoglobulin Fc region" refers to a region including a heavy chain constant domain 2 (CH2) and/or a heavy chain constant domain 3 (CH3) excluding the heavy chain and light chain variable domains of the immunoglobulin. The immunoglobulin Fc region may be a component constituting a moiety of the conjugate of the present invention. Specifically, the immunoglobulin Fc region corresponds to F in Chemical Formula 1 above.

Throughout the specification, the Fc region includes not only a native sequence obtained from papain digestion of immunoglobulin but also derivatives thereof, e.g., a sequence different from the native sequence and obtained by modification of one or more amino acid residues by deletion, addition, non-conservative or conservative substitution, or a combination thereof.

The F (*e*.*g*., immunoglobulin Fc region) has a structure in which two polypeptide chains are linked to each other by a disulfide bond, wherein the linkage is formed by a nitrogen atom of only one of the two chains, but is not limited thereto. Linkage via the nitrogen atom may be formed by reductive amination of an ε-amino group or an N-terminal amino group of lysine.

The reductive amination refers to a reaction in which an amine group or an amino group of one reactant reacts with an aldehyde group (a functional group capable of participating reductive amination) of another reactor to produce an amine, and then an amine bond is formed by reduction, as an organic synthesis reaction well known in the art.

As a specific example of the GLP-2 long-acting conjugate of the present invention, the immunoglobulin Fc region may be linked to the linker via the nitrogen atom of the N-terminus thereof in the long-acting conjugate.

The immunoglobulin Fc region may include a hinge region in the heavy chain constant domain, but is not limited thereto.

In the present invention, the immunoglobulin Fc region may include a particular hinge sequence at the N-terminus.

As used herein, the term "hinge sequence" refers to a site located at a heavy chain and forming a dimer of the immunoglobulin Fc region via an inter disulfide bond.

In the present invention, the hinge sequence may be mutated to have one cysteine residue by deletion of a part of the hinge sequence including the following amino acid sequence, without being limited thereto.
Glu-Ser-Lys-Tyr-Gly-Pro-Pro-Cys-Pro-Ser-Cys-Pro (SEQ ID NO: 11).

The hinge sequence may include only one cysteine residue as the 8^{th} cysteine residue or 11^{th} cysteine residue of the hinge sequence of SEQ ID NO: 11 is deleted. The hinge sequence of the present invention may consist of 3 to 12 amino acids including only one cysteine residue, without being limited thereto. More specifically, the hinge sequence of the present invention may have a sequence as follows: Glu-Ser-Lys-Tyr-Gly-Pro-Pro-Pro-Ser-Cys-Pro (SEQ ID NO: 12), Glu-Ser-Lys-Tyr-Gly-Pro-Pro-Cys-Pro-Ser-Pro (SEQ ID NO: 13), Glu-Ser-Lys-Tyr-Gly-Pro-Pro-Cys-Pro-Ser (SEQ ID NO: 14), Glu-Ser-Lys-Tyr-Gly-Pro-Pro-Cys-Pro-Pro (SEQ ID NO: 15), Lys-Tyr-Gly-Pro-Pro-Cys-Pro-Ser (SEQ ID NO: 16), Glu-Ser-Lys-Tyr-Gly-Pro-Pro-Cys (SEQ ID NO: 17), Glu-Lys-Tyr-Gly-Pro-Pro-Cys (SEQ ID NO: 18), Glu-Ser-Pro-Ser-Cys-Pro (SEQ ID NO: 19), Glu-Pro-Ser-Cys-Pro (SEQ ID NO: 20), Pro-Ser-Cys-Pro (SEQ ID NO: 21), Glu-Ser-Lys-Tyr-Gly-Pro-Pro-Ser-Cys-Pro (SEQ ID NO: 22), Lys-Tyr-Gly-Pro-Pro-Pro-Ser-Cys-Pro (SEQ ID NO: 23), Glu-Ser-Lys-Tyr-Gly-Pro-Ser-Cys-Pro (SEQ ID NO: 24), Glu-Ser-Lys-Tyr-Gly-Pro-Pro-Cys (SEQ ID NO: 25), Lys-Tyr-Gly-Pro-Pro-Cys-Pro (SEQ ID NO: 26), Glu-Ser-Lys-Pro-Ser-Cys-Pro (SEQ ID NO: 27), Glu-Ser-Pro-Ser-Cys-Pro (SEQ ID NO: 28), Glu-Pro-Ser-Cys (SEQ ID NO: 29), or Ser-Cys-Pro (SEQ ID NO: 30).

More specifically, the hinge sequence may include an amino acid sequence of SEQ ID NO: 21 (Pro-Ser-Cys-Pro) or SEQ ID NO: 30 (Ser-Cys-Pro), without being limited thereto.

In a more specific embodiment of the GLP-2 derivative long-acting conjugate of the present invention, the N-terminus of the immunoglobulin Fc region is proline in the conjugate and the immunoglobulin Fc region is linked to the linker by a nitrogen atom of the proline in the conjugate.

In an embodiment of the GLP-2 derivative long-acting conjugate of the present invention, the immunoglobulin Fc region may be in the form of a dimer in which two immunoglobulin Fc region chains form a homodimer or heterodimer by the hinge sequence. The conjugate of Chemical Formula 1 of the present invention may be in a form in which one end of the linker is linked to one of the two immunoglobulin Fc region chains, without being limited thereto.

As used herein, the term "N-terminus" refers to an amino terminus of a protein or polypeptide and may include the last amino acid residue located at the end of the amino terminus or 1, 2, 3, 4, 5, 6, 7, 8, 9, 10 or more amino acids from the end of the amino terminus. The immunoglobulin Fc region of the present invention may include the hinge sequence at the N-terminus, without being limited thereto.

Also, the immunoglobulin Fc region of the present invention may be an extended Fc region including a part of or the entirety of a heavy chain constant domain 1 (CH1) and/or a light chain constant domain 1 (CL1) excluding the heavy chain and the light chain variable domains of the immunoglobulin, as long as the immunoglobulin Fc region has substantially identical or enhanced effects compared to the native type. Also, the immunoglobulin Fc region may be a region from which a considerably long part of the amino acid sequence corresponding to the CH2 and/or CH3 is removed.

For example, the immunoglobulin Fc region of the present invention may include 1) CH1 domain, CH2 domain, CH3 domain, and CH4 domain, 2) CH1 domain and CH2 domain, 3) CH1 domain and CH3 domain, 4) CH2 domain and CH3 domain, 5) a combination of one or more domains selected from CH1 domain, CH2 domain, CH3 domain, and CH4 domain and an immunoglobulin hinge region (or a part of the hinge region), or 6) a dimer of each domain of the heavy chain constant domain and the light chain constant domain, but is not limited thereto.

Also, as a specific example, the immunoglobulin Fc region may be in a dimeric form and one X molecule is covalently linked to one immunoglobulin Fc region in the dimer form. In this case, the immunoglobulin Fc may be linked to X via the non-peptidyl polymer. Meanwhile, two X molecules may also be symmetrically linked to one immunoglobulin Fc region in the dimeric form. In this case, the immunoglobulin Fc region may be linked to X via the non-peptidyl linker. However, the embodiment is not limited to the above-described example.

In addition, the immunoglobulin Fc region of the present invention includes not only a native amino acid sequence but also amino acid sequence derivatives thereof. The amino acid sequence derivative refers to an amino acid sequence having a difference in at least one amino acid residue by deletion, addition, non-conservative or conservative substitution, or a combination thereof.

For example, in the case of IgG Fc, amino acid residues at positions 214 to 238, 297 to 299, 318 to 322 or 327 to 331, which are known to be important in the linkage, may be used as suitable sites for alteration.

Also, various other types of derivatives may be prepared, for example, by removing a region capable of forming a disulfide bond, removing several amino acids from the N-terminus of the native Fc, or adding a methionine residue to the N-terminus of the native Fc. Also, to remove effector functions, a complement-binding site, e.g., a C1q-binding site, may be removed, or an antibody dependent cell mediated cytotoxicity (ADCC) site may be removed. Techniques of preparing these sequence derivatives of the immunoglobulin Fc region are disclosed in International Patent Publication Nos. WO 97/34631 and WO 96/32478, *etc.*

Amino acid exchanges in proteins and peptides, which do not alter the activity thereof are well known in the art (H. Neurath, R. L. Hill, *The Proteins*, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. If required, the Fc region may be modified by phosphorylation, sulfation, acrylation, glycosylation, methylation, farnesylation, acetylation, amidation, and the like.

The above-described Fc derivatives may have biological activity identical to that of the Fc region of the present invention and improved structural stability against heat, pH, or the like.

In addition, the Fc region may be obtained from native forms isolated *in vivo* from humans or animals such as cows, goats, pigs, mice, rabbits, hamsters, rats, or guinea pigs or may be recombinants or derivatives thereof, obtained from transformed animal cells or microorganisms. In this regard, the Fc region may be obtained from a native immunoglobulin by isolating whole immunoglobulin from a living body of a human or animal and treating the isolated immunoglobulin with protease. When the whole immunoglobulin is treated with papain, it is cleaved into Fab and Fc regions, whereas when the whole immunoglobulin is treated with pepsin, it is cleaved into pF'c and F(ab)₂ fragments. Fc or pF'c may be isolated therefrom using size-exclusion chromatography, or the like. In a more specific embodiment, a human-derived Fc region is a recombinant immunoglobulin Fc region obtained from a microorganism.

In addition, the immunoglobulin Fc region may have native glycans or increased or decreased glycans compared to the native type, or be in a deglycosylated form. The increase, decrease, or removal of glycans of the immunoglobulin Fc may be achieved by any methods commonly used in the art such as a chemical method, an enzymatic method, and a genetic engineering method using a microorganism. In this regard, the immunoglobulin Fc region obtained by removing glycans shows a significant decrease in binding affinity to a complement c1q and a decrease in or loss of antibody-dependent cytotoxicity or complement-dependent cytotoxicity, and thus unnecessary immune responses are not induced thereby in living organisms. Based thereon, a deglycosylated or aglycosylated immunoglobulin Fc region may be more suitable as a drug carrier in view of the objects of the present invention.

As used herein, the term "deglycosylation" refers to a Fc region from which glycan is removed using an enzyme and the term "aglycosylation" refers to a Fc region that is not glycosylated and produced in prokaryotes, more specifically *E*. *coli.*

Meanwhile, the immunoglobulin Fc region may be derived from humans or animals such as cows, goats, pigs, mice, rabbits, hamsters, rats, or guinea pigs. In a more specific embodiment, the immunoglobulin Fc region may be derived from humans.

In addition, the immunoglobulin Fc region may be derived from IgG, IgA, IgD, IgE, or IgM, or any combination or hybrid thereof. In a more specific embodiment, it is derived from IgG or IgM which are the most abundant proteins in human blood, and in an even more specific embodiment, it is derived from IgG known to enhance the half-lives of ligand-binding proteins. In an even more specific embodiment, the immunoglobulin Fc region is an IgG4 Fc region, and in the most specific embodiment, the immunoglobulin Fc region is an aglycosylated Fc region derived from human IgG4, without being limited thereto.

In addition, in a specific embodiment, the immunoglobulin Fc fragment may be a human IgG4 Fc fragment in the form of a homodimer in which two monomers are linked to each other via a disulfide bond (inter-chain) formed between cysteines that are the 3^{rd} amino acids of each monomer. In this regard, the homodimer has or may have independent two inner disulfide bonds (intra-chain), *i*.*e*., a disulfide bond formed between 35^{th} and 95^{th} cysteines and a disulfide bond formed between 141^{st} and 199^{th} cysteines. Each monomer may consist of 221 amino acids, but is not limited thereto.

Because each monomer consists of 221 amino acids, the number of amino acids constituting the homodimer may be 442 in total, without being limited thereto. Specifically, the immunoglobulin Fc fragment may be in the form of a homodimer in which two monomers each having an amino acid sequence of SEQ ID NO: 31 (consisting of 221 amino acids) are linked to each other via a disulfide bond between cysteines that are 3^{rd} amino acids, wherein the monomers of the homodimer each independently have a disulfide bond formed between 35^{th} and 95^{th} cysteines and a disulfide bond formed between 141^{st} and 199^{th} cysteines, without being limited thereto.

Meanwhile, as used herein, the term "combination" refers to formation of a linkage between a polypeptide encoding a single-chain immunoglobulin Fc region of the same origin and a single-chain polypeptide of a different origin when a dimer or a multimer is formed. That is, a dimer or multimer may be prepared using two or more Fc fragments selected from the group consisting of IgG Fc, IgA Fc, IgM Fc, IgD Fc, and IgE Fc fragments.

As used herein, the term "hybrid" means that sequences corresponding to two or more immunoglobulin Fc fragments of different origins are present in a single-chain of an immunoglobulin constant region. In the present invention, various hybrid forms are possible. That is, a domain hybrid may be composed of 1 to 4 domains selected from the group consisting of CH1, CH2, CH3, and CH4 of IgG Fc, IgM Fc, IgA Fc, IgE Fc, and IgD Fc and may further include a hinge region.

Meanwhile, IgG may also be divided into IgG1, IgG2, IgG3 and IgG4 subclasses, which may be combined or hybridized in the present invention. Specifically, IgG is divided into IgG2 and IgG4 subclasses, more specifically Fc fragments of IgG4 rarely having effector functions such as complement dependent cytotoxicity (CDC).

In addition, the above-described conjugate may have an improved long-lasting efficacy compared to the native GLP-2 or compared to X not modified with F, and the conjugate may include a form enclosed in biodegradable nanoparticles as well as those described above, without being limited thereto.

Meanwhile, the GLP-2 derivative and a long-acting conjugate thereof may include all of those in the form of the peptide or compound itself, a salt thereof (*e*.*g*., pharmaceutically acceptable salt of the peptide), or a solvate thereof.

In addition, the peptide, the compound, the GLP-2 derivative, or the long-acting conjugate thereof may be in any pharmaceutically acceptable form.

The type of the salt is not particularly limited. However, the salt is preferably in a form safe and effective to a subject, *e*.*g*., a mammal, without being limited thereto.

The term, "pharmaceutically acceptable" refers to a substance that may be effectively used for the intended use within the scope of pharmaco-medical decision without inducing excessive toxicity, irritation, allergic responses, and the like.

As used herein, the term "pharmaceutically acceptable salt" refers to a salt derived from a pharmaceutically acceptable inorganic acid, organic acid, or base. Examples of a suitable acid may include hydrochloric acid, bromic acid, sulfuric acid, nitric acid, perchloric acid, fumaric acid, maleic acid, phosphoric acid, glycolic acid, lactic acid, salicylic acid, succinic acid, toluene-*p*-sulfonic acid, tartaric acid, acetic acid, citric acid, methanesulfonic acid, formic acid, benzoic acid, malonic acid, naphthalene-2-sulfonic acid, and benzenesulfonic acid. Examples of the salt derived from a suitable base may include alkali metals such as sodium and potassium, alkaline earth metals such as magnesium, and ammonium.

In addition, as used herein, the term "solvate" refers to a complex of the peptide, the compound, or the salt thereof according to the present invention and a solvent molecule.

The GLP-2 derivative, the long-acting conjugate, and the composition comprising the same may be used to prevent, ameliorate, or treat mucositis induced by radiotherapy, chemotherapy, or a combination thereof.

As used herein, the term "prevention" refers to all actions intended to completely or partially inhibit or delay development of mucositis induced by radiotherapy, chemotherapy, or a combination thereof by administering the GLP-2 derivative, the long-acting conjugate of the GLP-2 derivative, and/or the composition comprising the same. The term "treatment" refers to all actions to completely or partially alleviate or beneficially change symptoms of mucositis induced by radiotherapy, chemotherapy, or a combination thereof and includes reduction or alleviation of symptoms of mucositis, alleviation of pain of the symptoms, decrease in incidence of mucositis, other changes in patients to enhance effects of treatment, or the like.

As used herein, the term "administration" refers to introduction of a particular substance into a patient by an appropriate method, and an administration route of the composition may be any conventional route that enables delivery of the composition to the target in living organisms, for example, via an oral administration route or a parenteral administration route such as an intradermal, intravenous, intramuscular, intraarterial, intramedullary, intrathecal, intraventricular, intrapulmonary, transdermal, subcutaneous, intraperitoneal, intranasal, intestinal, topical, sublingual, vaginal, or rectal route, without being limited thereto. However, since peptides are digested upon oral administration, active ingredients of a composition for oral administration should be coated or formulated for protection against degradation in the stomach. Preferably, the present composition may be administered in an injectable form. In addition, in the present invention, the composition may be administered using a certain device capable of delivering the active ingredients into a target cell.

In a specific embodiment, the composition of the present invention may be administered together with radiotherapy and/or chemotherapy concurrently, sequentially, or in reverse order. Specifically, the composition of the present invention may be administered before radiotherapy and/or chemotherapy is conducted (e.g., within 1 day, 2 days, 3 days, 4 days, 5 days, or 6 days; or one week or one month before radiotherapy and/or chemotherapy is conducted), but is not limited thereto. In addition, the composition of the present invention may be administered after radiotherapy and/or chemotherapy is conducted (e.g., within 1 day, 2 days, 3 days, 4 days, 5 days, or 6 days; one week or one month; or at the 1^{st} day (24 hours)) after radiotherapy and/or chemotherapy is conducted, without being limited thereto.

The pharmaceutical composition of the present invention may further include a pharmaceutically acceptable carrier, excipient, or diluent. The pharmaceutically acceptable carrier, excipient, or diluent may be one that does not occur naturally.

As used herein, the term "pharmaceutically acceptable" refers to an amount sufficient for exhibiting therapeutic effects without causing side effects and may be easily determined based on factors well known in the medical field such as the type of disease, age, body weight, health status, gender, and sensitivity to drug of a patient, administration route, administration method, the number of administration, duration of treatment, and a drug used in combination or concurrently.

The pharmaceutical composition of the present invention may further include the pharmaceutically acceptable carrier. For oral administration, the pharmaceutically acceptable carrier may contain a binder, a lubricant, a disintegrator, an excipient, a solubilizer, a dispersant, a stabilizer, a suspending agent, a coloring agent, a flavoring agent, and the like, without being limited thereto. For injectable preparations, the pharmaceutically acceptable carrier may contain a buffer, a preservative, an analgesic, a solubilizer, an isotonic agent, a stabilizer, and the like. For preparations for topical administration, the pharmaceutically acceptable carrier may contain a base, an excipient, a lubricant, a preservative, and the like.

The composition of the present invention may be formulated into various forms in combination with the above-mentioned pharmaceutically acceptable carriers. For example, for oral administration, the pharmaceutical composition may be formulated into tablets, troches, capsules, elixirs, suspensions, syrups, wafers, and the like. For injectable preparations, the pharmaceutical composition may be formulated into a single-dose ampoule or multidose form. The pharmaceutical composition may also be formulated into solutions, suspensions, tablets, pills, capsules, sustained-release preparations, and the like.

Meanwhile, examples of the carrier, the excipient, or the diluent suitable for formulations may include lactose, dextrose, sucrose, sorbitol, mannitol, xylitol, erythritol, maltitol, starch, Acacia rubber, alginate, gelatin, calcium phosphate, calcium silicate, cellulose, methyl cellulose, amorphous cellulose, polyvinyl pyrrolidone, water, methyl hydroxy benzoate, propylhydroxy benzoate, talc, magnesium stearate and mineral oils. Also, the pharmaceutical composition may further include a filler, an anti-coagulant, a lubricant, a humectant, a flavoring agent, a preservative, and the like.

In addition, the pharmaceutical composition of the present invention may be formulated in a formulation selected from the group consisting of tablets, pills, powders, granules, capsules, suspensions, formulations for internal use, emulsions, syrups, sterilized aqueous solutions, non-aqueous solvents, lyophilized preparations, and suppositories.

In addition, the composition may be formulated in a unit dosage form suitable for administration into the body of a patient, specifically in a form useful for administration of protein medicines according to a method commonly used in the medical field and administered using an administration method commonly used in the art, without being limited thereto.

In addition, the GLP-2 derivative or a long-acting conjugate thereof may be used in combination with various carriers permitted as medicaments such as a saline solution or an organic solvent. As the medicaments, carbohydrates such as glucose, sucrose, or dextran, antioxidants such as ascorbic acid or glutathione, chelating agents, low-molecular weight proteins, or other stabilizers may be used to improve stability or absorbability.

A dosage and number of administration of the pharmaceutical composition of the present invention are determined according to types of a drug, as an active ingredient, together with various related factors such as disease to be treated, administration route, age, gender, and body weight of a patient, and severity of the disease. Specifically, the composition of the present invention may include the GLP-2 derivative or the long-acting conjugate thereof in a pharmaceutically effective amount, without being limited thereto.

In the present invention, the pharmaceutically effective amount of the GLP-2 derivative or the long-acting conjugate thereof contained in the pharmaceutical composition refers to a level to obtain a desired pharmacological activity by using the GLP-2 derivative or the long-acting conjugate thereof, or a pharmaceutically acceptable level without causing toxicity or side effects or causing insignificant toxicity or side effects in a subject administered with the composition, without being limited thereto. The pharmaceutically effective amount may be determined in consideration of the number of administration, the patient, the formulation, and the like.

A total effective amount of the composition of the present invention may be administered to a patient in a single dose or in multiple doses using a fractionated treatment protocol for a prolonged period of time. The amount of an effective ingredient of the pharmaceutical composition of the present invention may vary according to severity of a disease. Specifically, a preferred daily dosage of the GLP-2 derivative or the long-acting conjugate thereof of the present invention may be from about 0.0001 mg to 500 mg, from about 0.001 mg to 100 mg, more preferably from about 0.01 mg to 10 mg per 1 kg of the body weight of the patient, without being limited thereto.

However, since the dosage of the GLP-2 derivative or the long-acting conjugate thereof is determined as an effective dosage for the patient in consideration of various factors such as age, body weight, health status, gender of the patient, severity of disease, diet, and excretion rate as well as route and number of administration of the pharmaceutical composition, an appropriate effective dosage for a particular use of the composition of the present invention may be determined by one of ordinary skill in the art by considering these factors. Formulation, administration route, and administration method of the pharmaceutical composition are not particularly limited as long as the effects of the present invention are obtained.

The pharmaceutical composition of the present invention has excellent *in vivo* persistence and potency, the number and frequency of administration of the pharmaceutical preparations according to the present invention may be significantly reduced.

Another aspect of the present invention provides a food composition for preventing or ameliorating mucositis induced by radiotherapy, chemotherapy, or a combination thereof, the food composition comprising the GLP-2 derivative and/or a long-acting conjugate thereof.

The GLP-2 derivative, the long-acting conjugate thereof, and mucositis induced by radiotherapy, chemotherapy, or a combination thereof are as described above.

The food composition may be used as a health functional food. When the composition of the present invention is used as a food aid additive, the peptide (peptide itself or a long-acting conjugate thereof) may be added alone or used with any other food or food ingredients appropriately according to any known method. The amount of the active ingredient to be mixed therewith may be appropriately determined according to purposes thereof (prevention, health, or treatment).

As used herein, the term "health functional food" refers to a food prepared using a particular ingredient as a raw material or a food manufactured or processed by extracting, concentrating, purifying, or mixing a particular ingredient contained in food and using the ingredient as a raw material for the purpose of health supplement. The health functional food is designed and processed to sufficiently exert body-regulatory functions, such as biodefense, biorhythm control, disease prevention, and recovery from disease, by the ingredient, and the health functional food composition may perform functions related to prevention of disease or recovery from disease.

Another aspect of the present invention provides a method for preventing, ameliorate, or treat mucositis induced by radiotherapy, chemotherapy, or a combination thereof, the method comprising administering the GLP-2 derivative, a long-acting conjugate thereof, and/or a composition comprising the same to a subject in need thereof.
the GLP-2 derivative, the long-acting conjugate thereof, the composition, and mucositis induced by radiotherapy, chemotherapy, or a combination thereof are as described above.

Specifically, the GLP-2 derivative may include an amino acid sequence represented by General Formula 1 above (wherein a sequence identical to SEQ ID NO: 1 is excluded from the amino acid sequence of General Formula 1), without being limited thereto.

In the present invention, the subject, as a subject suspected to have mucositis induced by radiotherapy, chemotherapy, or a combination thereof, may be a mammal such as rats and livestock including humans with the disease or at the risk of developing the disease and includes any subject that may be treated with the GLP-2 derivative, the long-acting conjugate thereof, and/or the pharmaceutical composition including the same of the present invention without limitation. In addition, the humans may be excluded from the subject of the present invention, without being limited thereto.

The method of the present invention may include administering the GLP-2 derivative, the long-acting conjugate thereof, and/or the composition comprising the same in a pharmaceutically effective amount. An appropriate daily dose may be determined by a doctor within the scope of sound medical judgment in a bolus or in multiple doses. However, in view of the objects of the present invention, it is preferred that a specific therapeutically effective amount for a particular patient is differently applied depending on various factors including the type and extent of a response to be achieved, a specific composition including whether other formulations are used according to the case, age, body weight, general health status, gender, and diet of the patient, administration time, administration route, excretion rate of the composition, duration of treatment, a drug used in combination or concurrently with the specific composition and similar factors well known in the medical field.

Another aspect of the present invention provides a prophylactic, ameliorative, or therapeutic use of the GLP-2 derivative, a long-acting conjugate thereof, and/or a pharmaceutical composition comprising the same for mucositis induced by radiotherapy, chemotherapy, or a combination thereof.

The GLP-2 derivative, the long-acting conjugate thereof, the composition, and mucositis induced by radiotherapy, chemotherapy, or a combination thereof are as described above.

Specifically, the GLP-2 derivative may include an amino acid sequence represented by General Formula 1 above (wherein a sequence identical to SEQ ID NO: 1 is excluded from the amino acid sequence of General Formula 1), without being limited thereto.

Another aspect of the present invention provides a use of the GLP-2 derivative, a long-acting conjugate thereof, and/or a pharmaceutical composition comprising the same in preparation of a drug for preventing or treating mucositis induced by radiotherapy, chemotherapy, or a combination thereof.

The GLP-2 derivative, the long-acting conjugate thereof, and mucositis induced by radiotherapy, chemotherapy, or a combination thereof are as described above.

Specifically, the GLP-2 derivative may include an amino acid sequence represented by General Formula 1 (wherein a sequence identical to SEQ ID NO: 1 is excluded from the amino acid sequence of General Formula 1), without being limited thereto.

Hereinafter, the present invention will be described in more detail with reference to the following examples. However, the following examples are merely presented to exemplify the present invention, and the scope of the present invention is not limited thereto.

### Example 1: Preparation of GLP-2 Derivative Long-acting Conjugate

For PEGylation of ALD(2) PEG, which is modified polyethylene glycol (in which hydrogen at both ends is substituted with a propionaldehyde group (3-oxopropyl group), and a formula weight of an ethyleneglycol repeating unit (moiety) was 3.4 kDa, NOF, Japan), into a GLP-2 derivative, CA GLP-2 RK having SEQ ID NO: 4 of Table 1, the GLP-2 derivative and ALD(2) PEG were reacted at a molar ratio of 1:5 to 1:20 with a GLP-2 derivative concentration of 5 mg/mL to 10 mg/mL at a temperature of 2°C to 8°C for 4 to 16 hours. At this time, the reaction was conducted in 20 mM HEPES at pH 7.5, in ethanol with addition of 20 mM sodium cyanoborohydride as a reducing agent. The reaction solution was purified with a Source 15S column (GE, USA) using a buffer solution containing sodium citrate (pH 2.0) and ethanol and a potassium chloride concentration gradient to thereby purify the mono-PEGylated GLP-2 derivative.

Subsequently, the purified mono-PEGylated GLP-2 derivative and the immunoglobulin Fc fragment of SEQ ID NO: 32 were reacted at a molar ratio of 1:2 to 1:6 with a total protein concentration of 30 mg/mL to 35 mg/mL at a temperature of 2°C to 8°C for 12 to 20 hours. At this time, the reaction solution contained a 100 mM calcium phosphate buffer (pH 6.0) and isopropanol with addition of 20 mM sodium cyanoborohydride as a reducing agent.

Upon completion of the reaction, the reaction solution was purified with a Source15Q column (GE, USA) using a bis-Tris buffer (pH 6.5) and a sodium chloride concentration gradient and with a Source 15ISO (GE, USA) using ammonium sulfate and a sodium citrate concentration gradient (pH 5.0 to 5.2) to thereby purify a long-acting conjugate of the GLP-2 derivative that is a conjugate in which the GLP-2 derivative is linked to the immunoglobulin Fc fragment via a covalent bond by the polyethyleneglycol linker.

### Example 2: Confirmation of Increased Small Intestine Mass in Rat Model in which Gastrointestinal Mucositis is Induced by Chemotherapy by Administering GLP-2 Derivative Long-acting Conjugate

In order to measure effects of the GLP-2 derivative long-acting conjugate prepared in Example 1 on ameliorating the small intestine *in vivo,* rats in which gastrointestinal mucositis was induced by chemotherapy (Docetaxel + Cyclophosphamide, TC) were used.

Specifically, 7-week-old male rats (SD rat) were divided into groups (3 rats per each group): a normal control (not treated with chemotherapy, G1, (SHarm) Vehicle), a control having gastrointestinal mucositis induced by chemotherapy (G2, (TC) vehicle), and a group in which gastrointestinal mucositis was induced by chemotherapy and then treated with the GLP-2 derivative long-acting conjugate prepared in Example 1 (3.1 mg/kg/QW (0.5 mg/kg/QM HED)) (G3, (TC) long-acting conjugate of the GLP-2 derivative) (in the present invention, the numerical value as the dose of the GLP-2 derivative long-acting conjugate is a value obtained by subtracting a mass of the polyethyleneglycol linker from a total mass of the used GLP-2 derivative long-acting conjugate, i.e., a sum of masses of polypeptide regions, as a reference). Gastrointestinal mucositis was induced in G2 and G3 by administering Docetaxel (36 mg/kg) and Cyclophosphamide (300 mg/kg) (D0). On the next day (D1), after each group was subcutaneously administered with the vehicle or the GLP-2 derivative long-acting conjugate alone, small intestine mass thereof was measured on D2, D3, and D4.

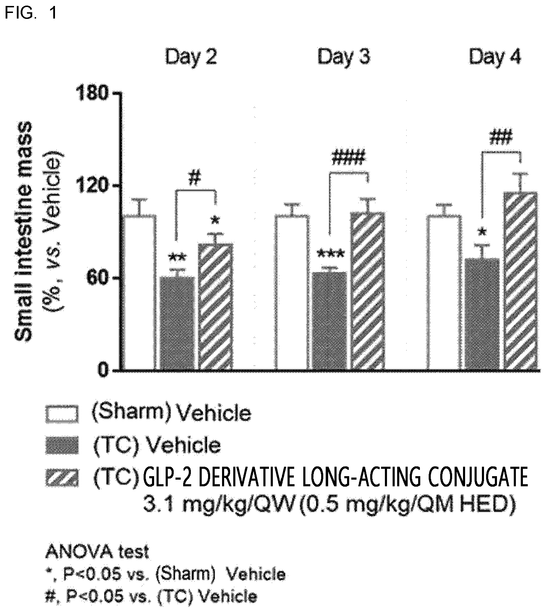

As a result, it was confirmed that the small intestine mass was significantly recovered within a short period of time in the group administered with the long-acting conjugate of the GLP-2 derivative compared to the control having gastrointestinal mucositis induced by chemotherapy (FIG. 1).

These results indicate that the GLP-2 derivative long-acting conjugate of the present invention has prophylactic and therapeutic effects on gastrointestinal mucositis induced by chemotherapy by rapidly recovering the small intestine.

### Example 3: Confirmation of Changed Small Intestine Mass in Rat Model in which Gastrointestinal Mucositis is Induced by Chemotherapy by Administering GLP-2 Derivative and GLP-2 Derivative Long-acting Conjugate

In order to measure effects of the GLP-2 derivative and the GLP-2 derivative long-acting conjugate prepared in Example 1 on ameliorating the small intestine in *vivo*, rats in which gastrointestinal mucositis was induced by chemotherapy (Docetaxel + Cyclophosphamide, TC) were used. Teduglutide was selected as a representative example of the GLP-2 derivative and used for experiments.

Specifically, 7-week-old male rats (SD rat) were divided into groups (5 rats per each group): a normal control (not treated with chemotherapy G1, Vehicle), a control having gastrointestinal mucositis induced by chemotherapy (G2, (TC) vehicle), a group administered with a GLP-2 derivative (Teduglutide 0.1 mg/kg/BID, Eₘₐₓ) simultaneously (D0) inducing gastrointestinal mucositis by chemotherapy (G3, (TC) Teduglutide (D0)), a group administered with the GLP-2 derivative long-acting conjugate prepared in Example 1 (3.1 mg/kg/QW (0.5 mg/kg/QM HED)) simultaneously (D0) inducing gastrointestinal mucositis by chemotherapy (G4, (TC) GLP-2 derivative long-acting conjugate (D0)), and a group administered with the GLP-2 derivative long-acting conjugate prepared in Example 1 (3.1 mg/kg/QW (0.5 mg/kg/QM HED)) one day before (D-1) inducing gastrointestinal mucositis by chemotherapy (G5, (TC) GLP-2 derivative long-acting conjugate (D-1)). In addition, the groups in which gastrointestinal mucositis was induced by chemotherapy were administered with Docetaxel (36 mg/kg) and Cyclophosphamide (300 mg/kg).

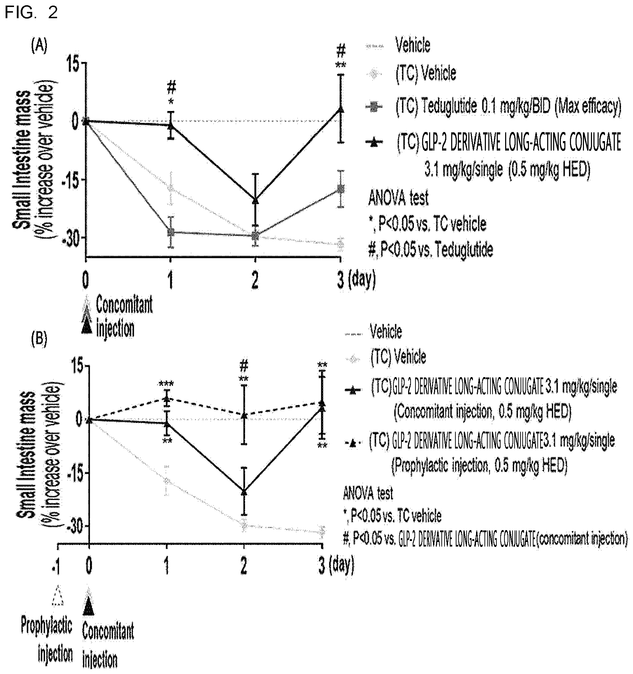

As a result, it was confirmed that the small intestine mass reduction level by gastrointestinal mucositis induced by chemotherapy was significantly lower, and the small intestine mass increase level by recovering was significantly higher in the group administered with the GLP-2 derivative long-acting conjugate (G4) compared to the group administered with the GLP-2 derivative (G3) (FIG. 2, A). In addition, upon comparison between the group administered with the GLP-2 derivative long-acting conjugate one day before (D-1) inducing gastrointestinal mucositis by chemotherapy (G5, (TC) long-acting conjugate of the GLP-2 derivative (D-1)) and the group administered with the GLP-2 derivative long-acting conjugate simultaneously (D0) inducing gastrointestinal mucositis by chemotherapy (G4, (TC) GLP-2 derivative long-acting conjugate (D0)), it was confirmed that the small intestine mass reduction due to chemotherapy was significantly lower in the case of administering the GLP-2 derivative long-acting conjugate one day before inducing gastrointestinal mucositis by chemotherapy (FIG. 2, B).

These results shows that administration of the GLP-2 derivative long-acting conjugate of the present invention has excellent prophylactic or therapeutic effects on gastrointestinal mucositis induced by chemotherapy by preventing damage to the small intestine and rapidly recovering from damage compared to administration of the GLP-2 derivative, indicating that administration of the GLP-2 derivative long-acting conjugate has higher prophylactic effects when the administration is conducted one day before chemotherapy.

### Example 4: Confirmation of Effects of GLP-2 Derivative Long-acting Conjugate on Inhibiting M1 Polarization, Inhibiting Macrophage Differentiation, and Inhibiting Monocyte Migration in THP-1 Cell (Monocyte)

In order to confirm the effects of the GLP-2 derivative long-acting conjugate prepared in Example 1 on inhibiting M1 polarization, inhibiting macrophage differentiation, and inhibiting monocyte migration in THP-1 cells (Monocytes), *in vitro* experiments were conducted as follows.

### [Experiment on Inhibition of M1 Polarization]

THP-1 cells were treated with the GLP-2 derivative long-acting conjugate prepared in Example 1 (10 µM) for 4 hours. The cells were then treated with lipopolysaccharides (LPS) at a concentration of 1 µg/mL for 2 hours to induce inflammation. Then, RNAs were extracted, and expression levels of mRNAs of TNF-α, IL-1β, and IL-6, which are pro-inflammatory cytokines (M1 polarization), were measured using qPCR.

### [Experiment on Inhibition of Macrophage Differentiation]

THP-1 cells were treated with the GLP-2 derivative long-acting conjugate prepared in Example 1 (10 µM) simultaneously with phorbol 12-myristate 13-acetate (PMA) to induce differentiation of the THP-1 cells for 48 hours. Then, adherent cells that are differentiated into macrophages were counted.

### [Experiment on Inhibition of Monocyte Migration]

THP-1 cells were treated with the GLP-2 derivative long-acting conjugate prepared in Example 1 (10 µM) for 48 hours. Subsequently, the THP-1 cells treated with the drug were transferred to an upper chamber of a Boyden chamber, and CCL-2 (50 ng/mL) was added thereto to induce migration thereof to a bottom chamber, and then the cells were cultured for 4 hours. Then, the degree of migration was measured using a migration assay kit (abcam).

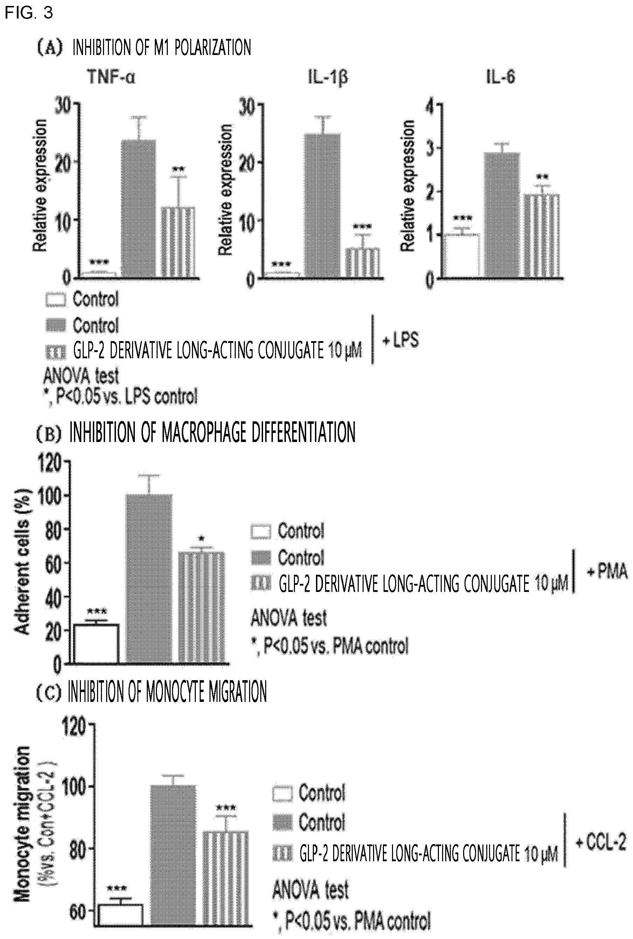

Through the experiments, the effects of the GLP-2 derivative long-acting conjugate on inhibiting M1 polarization (FIG. 3, A), inhibiting macrophage differentiation (FIG. 3, B), and inhibiting monocyte migration (FIG. 3, C).

These results indicate that the GLP-2 derivative long-acting conjugate of the present invention has prophylactic or therapeutic effects on gastrointestinal mucositis by inhibiting inflammation, inhibiting monocytes from differentiating into macrophages that cause inflammation, and inhibiting migration of monocytes to an inflammation site.

The above description of the present invention is provided for the purpose of illustration, and it would be understood by those skilled in the art that various changes and modifications may be made without changing the technical conception and essential features of the present invention. Thus, it is clear that the above-described embodiments are illustrative in all aspects and do not limit the present invention. Therefore, the scope of the disclosure is defined not by the detailed description, but by the claims and their equivalents, and all variations within the scope of the claims and their equivalents are to be construed as being included in the disclosure.

## Claims

1. A pharmaceutical composition for preventing or treating mucositis induced by radiotherapy, chemotherapy, or a combination thereof, the pharmaceutical composition comprising:
a pharmaceutically acceptable excipient; and
a glucagon-like peptide-2 (GLP-2) derivative in a pharmaceutically effective amount, wherein the GLP-2 derivative comprises an amino acid sequence represented by General Formula 1 below:
[General Formula 1] X₁X₂DGSFSDEMNTILDNLAARDFINWLIQTX₃₀ITDX₃₄ (SEQ ID NO: 10)
wherein
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or 2-aminoisobutyric acid (Aib);
X₃₀ is lysine or arginine;
X₃₄ is at least one amino acid or at least one altered amino acid; and
with the proviso that a sequence identical to SEQ ID NO: 1 is excluded from the amino acid sequence of General Formula 1.

2. The pharmaceutical composition according to claim 1, wherein the GLP-2 derivative comprises an amino acid sequence represented by General Formula 2 below:
[General Formula 2] X₁X₂DGSFSDEMNTILDNLAARDFINWLIQTX₃₀ITDX₃₄ (SEQ ID NO: 9)
wherein
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or 2-aminoisobutyric acid (Aib);
X₃₀ is lysine or arginine;
X₃₄ is absent, or lysine, arginine, glutamine, histidine, 6-azidolysine, or cysteine; and
with the proviso that a sequence identical to SEQ ID NO: 1 is excluded from the amino acid sequence of General Formula 2.

3. The pharmaceutical composition according to claim 2, wherein in the GLP-2 derivative,
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine.

4. The pharmaceutical composition according to claim 1, wherein the GLP-2 derivative comprises an amino acid sequence selected from the group consisting of SEQ ID NOS: 2 to 8.

5. The pharmaceutical composition according to claim 1, wherein a C-terminus of the GLP-2 derivative is non-altered or amidated.

6. The pharmaceutical composition according to claim 1, wherein the mucositis is oral mucositis, gastrointestinal mucositis, or a combination thereof.

7. The pharmaceutical composition according to claim 1, wherein the chemotherapy is chemotherapy using an anticancer drug.

8. The pharmaceutical composition according to claim 7, wherein the anticancer drug is a cytotoxic anticancer agent, a targeted anticancer agent, an immuno-oncology agent, or a combination thereof.

9. The pharmaceutical composition according to claim 8, wherein the cytotoxic anticancer agent is a nucleoside analogue, an antifolate, an antimetabolite, a topoisomerase I inhibitor, an anthracycline, a podophyllotoxin, a taxane, a vinca alkaloid, an alkylating agent, a platinum compound, or a combination thereof.

10. The pharmaceutical composition according to claim 7, wherein the anticancer drug is 5-fluorouracil (5-FU), cyclophosphamide (CPA), Docetaxel, Doxorubicin, Vincristine, Prednisone, Etoposide, ifosfamide, Methotrexate, Paclitaxel, Glemcitabine, Vinorelbine, Leucovorin, Irinotecan, Oxaliplatin, or a combination thereof.

11. The pharmaceutical composition according to claim 1, wherein the composition is administered within 1 day before conducting radiotherapy or chemotherapy; or within 1 day after conducting radiotherapy or chemotherapy.

12. The pharmaceutical composition according to claim 1, wherein the composition leads to at least one of an increase in small intestine mass, a decrease in a small intestine mass reduction level, an inhibition of inflammation, an inhibition of differentiation of monocytes into macrophages, and an inhibition of migration of monocytes, in a subject administered with the composition.

13. The pharmaceutical composition according to any one of claims 1 to 12, wherein the GLP-2 derivative is in the form of a long-acting conjugate in which the GLP-2 derivative is linked to a biocompatible material capable of increasing an *in vivo* half-life of the GLP-2 derivative.

14. The pharmaceutical composition according to claim 13, wherein the conjugate is represented by Chemical Formula 1 below:
[Chemical Formula 1] X-La-F
wherein,
X is a GLP-2 derivative;
L is a linker comprising an ethyleneglycol repeating unit;
a is 0 or a natural number with the proviso that when a is 2 or more, each L is independent of the other;
F is an immunoglobulin Fc region; and
the "-" is a covalent bond.

15. The pharmaceutical composition according to claim 14, wherein the immunoglobulin Fc region is an aglycosylated IgG4 Fc region.

16. The pharmaceutical composition according to claim 14, wherein the F is a dimer consisting of two polypeptide chains, and one end of the L is linked to only one polypeptide chain of the two polypeptide chains.

17. The pharmaceutical composition according to claim 14, wherein the L is polyethylene glycol.

18. The pharmaceutical composition according to claim 14, wherein a formula weight of the ethyleneglycol repeating unit moiety in the L is in a range of 1 kDa to 100 kDa.
